# EUROPEAN PATENT APPLICATION

(11) **EP 1 321 149 A1**
(43) Date of publication of application: **25.06.2003**
(21) Application number: 02026703.5
(22) Date of filing: 29.11.2002
(51) Int. Cl.: A61K 35/78, A61P 5/00

(54) **Compositions for relieving symptoms of menopausal syndrome**

(30) Priority: 18.12.2001 IT MI20012678
(71) Applicant: Marfarma S.R.L., 20145 Milano (IT)
(72) Inventor: Heyda, Alessandro, 20145 Milano (IT)
(74) Representative: Minoja, Fabrizio, Dr.

(57) **Abstract**

Disclosed are pharmaceutical, medical, dietetic, nutritional or cosmetic compositions based on soy isoflavones and/or black cohosh (*Cimicifuga racemosa*) and plant extracts with a neurotonic, neurosedative, antispasmodic, hypnotic and analgesic effect designed to alleviate the symptoms of menopausal syndrome.

## Description

The present invention relates to pharmaceutical, medical, dietetic, nutritional or cosmetic compositions containing soy isoflavones, extract of black cohosh and extracts of plants with a neurotonic action, especially *Griffonia simplicifolia,* which are useful for the treatment of the symptoms associated with menopausal syndrome.

### INTRODUCTION

It is widely recognised that the symptoms associated with the menopause, including neurovegetative symptoms (hot flushes, sweating, headache, etc.), psychological symptoms (irritability, depression, sleep disorders, etc.) and osteoporosis, which is often manifested in menopausal women, are attributable to oestrogen deficiency, and that hormone replacement treatment is consequently an effective therapeutic remedy.

However, oestrogen administration is considered risky, especially in the long term, due to the possible onset of oestrogen-dependent tumours. The administration of oestrogen-like substances of plant origin known as phytoestrogens, i.e. heterocyclic compounds contained in legumes and cereals which have a structure resembling that of oestradiol, is therefore preferable. The finding that oriental women, whose diet is rich in soy, present a low incidence of breast tumours and osteoporosis and infrequently suffer from the typical post-menopausal symptoms, especially nocturnal hot flushes, has led to the discovery that the isoflavones contained in soy, namely genistein and daidzein, especially genistein, act as bland agonists of the oestrogen receptor. Supplementation of the diet with soy derivatives or administration of the phytoestrogens deriving therefrom consequently represents a promising alternative to oestrogen administration.

Another source of phytoestrogens is black cohosh, a member of the Ranunculaceae family, whose roots and rhizome contain the natural oestrogen formononetin in addition to triterpene glycosides, cimicifugin and other substances. The extract, usually titrated in triterpene glycosides with values of between 0.5 and 30%, has proved effective at relatively low doses, usually not exceeding 40 mg a day, for the treatment of the neurovegetative disorders associated with the menopause and dysmenorrhoea, probably due to inhibition of LH (luteinising hormone) [E. Ducker et al., Planta Med., 57 (1991) 420] and inhibition of ovarian secretion of progesterone (H. Jarry et al., SL-95, Munich 1996). It has been proposed that the extract should be used in combination with compounds having an anti-oestrogen activity, especially tamoxifen, for the treatment of oestrogen-dependent tumours (US6267994).

The association of formononetin with other phytoestrogens such as those derived from soy, namely genistein and daidzein, has been described for the same use (US5830887), and for treatment of premenstrual syndrome and menopausal syndrome.

It has now been found that the beneficial effects of that association, in particular the reduction in hot flushes, are considerably boosted by extracts of plants with neurotonic activity, especially *Griffonia simplicifolia.*

### DESCRIPTION OF THE INVENTION

The present invention relates to compositions containing soy isoflavones and/or black cohosh extract in combination with plant extracts with a neurotonic, neurosedative, antispasmodic, hypnotic and analgesic effect. Examples of these plant extracts include extracts of stinking nightshade, passion-flower, lavender, yellow clover, deadly nightshade, hemlock, hawthorn, ginseng, ginkgo biloba, sweet clover, camomile of every genus and species, hops, linden, valerian, lemon balm, St. John's wort, yarrow, kava-kava, golden seal, elecampane, centella, nettle, woodruff, celery, couch grass, feverfew, poppies of every species and variety, sage, shepherd's purse and griffonia, preferably *Griffonia simplicifolia.* The corresponding active principle obtained by extraction or synthesis, or a derivative, metabolite or precursor thereof, can be used instead of the plant extract. *Griffonia simplicifolia* is a plant belonging to the legume family whose seeds (which contain the active principle) contain a significant amount of 5-HTP (5-hydroxytryptophan), the precursor of serotonin, a neurotransmitter responsible for controlling the sleep, appetite and mood functions. The active principle is consequently used as a dry extract in preparations with a slimming or antidepressant action or in energy products, together with other substances. The addition of *Griffonia simplicifolia* extract or its active principle or a derivative, precursor or metabolite thereof to soy isoflavones and/or black cohosh is particularly advantageous. As it acts on symptoms associated with anxiety and irritability, beneficial effects are produced on the manifestations of menopausal syndrome and post-menopausal symptoms, especially hot flushes.

Soy extracts with various isoflavone and saponin contents (cumulatively from 25 to 90%) are known and available on the market. The term "black cohosh extract" means the extract of the roots and rhizomes of *Cimicifuga racemosa*; the term "*Griffonia simplicifolia* extract" means the extract of the seeds contained in the pods.

Soy extracts with an isoflavone and saponin content of 5 to 90 % each and/or black cohosh extracts with a triterpene glycoside content of between 0.5% and 30% are used, together with *Griffonia simplicifolia* extracts having a 5-HTP (5-hydroxytryptophan) content ranging between 0.5 and 99%. As already stated, purified extracted or synthesised 5-HTP or a derivative, precursor or metabolite thereof can be used.

The compositions will preferably contain between 5 and 300 mg of soy isoflavones, between 5 and 900 mg of *Griffonia simplicifolia* extract expressed as 5-hydroxytryptophan, and between 5 and 900 mg of black cohosh extract, per administration unit.

The compositions of the present invention can be formulated in a way suited to oral, parenteral, transdermal or transmucosal administration in accordance with conventional techniques and excipients, as described in "Remington's Pharmaceutical Science Handbook", 17^{th} Ed. Mack Pub., N.Y., U.S.A..

Examples of such formulations are liquids and suspensions, injectable forms and sprays, aerosols, gels, emulsions, tablets, capsules, suppositories, transdermal plasters, granulates and powders. Gastro-protected and/or modified-release formulations are preferred for oral administration, especially modified-release tablets which release the active constituents 5-8 hours after administration, obtainable by means of polymers or copolymers conventionally used for the purpose, such as acrylic or methacrylic acid copolymers (Eudragit®) or cellulose derivatives. In the case of transdermal applications, an absorption promoter such as liposomes or a cross-linked polymer which traps the functional substances could also be used.

The example below illustrates the invention in greater detail.

### Example

Gastro-resistant tablet containing:

| | |
|---|---|
| soy extract | 400 mg |
| black cohosh extract | 40 mg |
| *Griffonia simplicifolia* extract | 375 mg |
| lactose | 245 mg |
| hydroxypropyl methylcellulose | 200 mg |

## Claims

1. Pharmaceutical, medical, dietetic, nutritional or cosmetic compositions containing soy isoflavones and/or black cohosh extract combined with plant extracts with a neurotonic, neurosedative, antispasmodic, hypnotic and analgesic effect and/or their active principle, or a derivative, precursor or metabolite thereof.

2. Compositions as claimed in claim 1 containing soy extracts corresponding to a quantity of isoflavones and saponins of between 5 and 300 mg each, depending on the administration unit.

3. Compositions as claimed in any one of the preceding claims containing 5 to 900 mg of black cohosh extract per administration unit.

4. Compositions as claimed in claim 1 wherein the plant extract with neurotonic, neurosedative, antispasmodic, hypnotic and analgesic action is *Griffonia simplicifolia.*

5. Compositions as claimed in claim 4 containing a quantity of *Griffonia simplicifolia* extract corresponding to 5-900 mg of 5-hydroxytryptophan.

6. Compositions as claimed in any one of claims 1 to 3, containing 5 to 900 mg of 5-hydroxytryptophan per administration unit or the corresponding amount of a derivative, precursor or metabolite thereof.

7. Compositions as claimed in any one of the preceding claims, in tablet form.

8. Compositions as claimed in claim 7, wherein the active constituents are released 5-8 hours after administration.
